# EUROPEAN PATENT APPLICATION

(11) **EP 3 108 923 A1**
(43) Date of publication of application: **28.12.2016**
(21) Application number: 15765279.3
(22) Date of filing: 16.03.2015
(51) Int. Cl.: A61M 37/00

(54) **MEDICINAL FLUID INJECTION DEVICE**

(30) Priority: 20.03.2014 KR 20140032458
(71) Applicant: Amorepacific Corporation, Seoul 140-777 (KR)
(72) Inventor: KWON, Min Kyung, Youngin-si Gyeonggi-do 446-729 (KR); JANG, Ji Hye, Youngin-si Gyeonggi-do 446-729 (KR); BAE, Joon Ho, Youngin-si Gyeonggi-do 446-729 (KR)
(74) Representative: Brevalex
(86) International application number: PCT/KR2015/002530
(87) International publication number: WO 2015/142013

(57) **Abstract**

The present invention relates to a medicinal fluid injection device for injecting a medicinal fluid into the skin of a patient so as to induce collagen generation and skin regeneration according to the radial diffusion of the medicinal fluid, comprising: a case part constituting the appearance; a medicinal fluid placing part mounted on the case part and in which a storage container storing a medicinal fluid is mounted; a medicinal fluid supply part connected to the storage container so as to supply a medicinal fluid; a medicinal fluid pressurization part mounted on the case part, and connected to the medicinal fluid supply part so as to pressurize the supplied medicinal fluid; and a medicinal fluid discharge part mounted on the medicinal fluid supply part so as to inject, into the skin, the medicinal fluid supplied from the medicinal fluid supply part. The medicinal fluid discharge part comprises: an outer case in which a plurality of needles are provided; and an inner case provided such that the front end is inserted into the outer case so as to be coupled thereto and the rear end is coupled to the medicinal fluid supply part. A through hole penetrating the inside and the outside of the inner case and forming a micro-jet from the medicinal fluid is formed at the front end of the inner case.

## Description

### Technical Field

The present invention relates to a medicinal fluid injection device, and more particularly, to a medicinal fluid injection device which injects medicinal fluid into the skin of a patient.

### Background Art

In medical procedures and cosmetic procedures, it is necessary to inject fluid under the skin. For instance, insulin is injected under the skin, or materials, such as collagen, are injected under the skin for the purpose of beauty.

The most general method for injecting fluid is to insert an injection needle into the desired area to the desired depth. However, the use of injection needle accompanies pain and bleeding and causes a problem that an unskilled user cannot properly insert the injection needle into the specific area or inserts the injection needle in mistake. Moreover, if the user inserts the injection needle into the skin in mistake, it may give a severe damage to the skin texture, and the damaged skin must get treatment for a considerably long period of time.

In order to solve the problems, recently, a needle-free syringe has been developed.

Such a needle-free syringe ejects fluid in the form of a microjet of high-pressure so that the fluid permeates into the skin in the form of the microjet. However, such a syringe must guarantee sufficient pressure and velocity in order to infiltrate the microjet into the skin to the desired depth, and the microjet penetrating into the skin must spread promptly within tissues. However, skins, especially, dead skin cells and the epidermis, are different from each other in thickness, elasticity, flexibility and intensity and are different according to areas even in the same object.

Therefore, it is difficult that the syringe infiltrates fluid evenly, and the syringe is also difficult to control loss of medicinal fluid and penetration depth when the medicinal fluid is injected because the angle and the distance between an orifice, from which the microjet is discharged, and the surface of the skin and material properties of the medicinal fluid to be infiltrated have a great effect on the infiltrated amount and depth of the medicinal fluid. Particularly, the conventional needle-free syringe has several disadvantages in that some of the infiltrated microjet does not spread within the skin but juts out from the skin in reverse because the microjet with a sufficient velocity is not formed when medicinal fluid of high-viscosity is transferred, and in that the syringe lowers skin penetration capacity because the medicinal fluid further reduces the velocity of the microjet while colliding against the microjet, which gets out of the orifice, in the reverse direction.

### Disclosure

### Technical Problem

Accordingly, the present invention has been made in an effort to solve the above-mentioned problems occurring in the prior arts, and it is an object of the present invention to provide a medicinal fluid injection device which can effectively transfer a microjet into the skin, minimize pain at the skin and bleeding when medicinal fluid permeates into the skin, and minimize an injection loss rate of the microjet.

It is another object of the present invention to provide a medicinal fluid injection device which can transfer medicinal fluid uniformly with no loss of the medicinal fluid without regard to material properties of the medicinal fluid and characteristics of the skin.

It is a further object of the present invention to provide a medicinal fluid injection device having an improved structure to endure high pressure applied to the medicinal fluid.

### Technical Solution

To achieve the above objects, the present invention provides a medicinal fluid injection device, which injects medicinal fluid into the skin in such a way as to radially spread the medicinal fluid so as to promote collagen generation of the skin, including: a case part which forms an outward appearance; a medicinal fluid placing part, which is mounted on the case part, and, to which a storage container storing medicinal fluid therein is mounted; a medicinal fluid supplying which is connected with the storage container to supply the medicinal fluid; a medicinal fluid pressurization part which is mounted on the case part and is connected to the medicinal fluid supply part to pressurize the supplied medicinal fluid; and a medicinal fluid discharge part which is mounted on the medicinal fluid supply part to inject the medicinal fluid supplied from the medicinal fluid supply part into the skin.

The medicinal fluid discharge part includes: an outer case on which a plurality of needles are mounted; and an inner case of which a front end portion is inserted and combined into the outer case and a rear end portion is combined and mounted to the medicinal fluid supply part; and a through hole is formed at the front end portion of the inner case in such a way as to permeate through the inner case in order to form a microjet.

The medicinal fluid discharge part further includes a spacing part which is detachably mounted at a front end portion of the outer case in order to uniformly maintain a distance between the medicinal fluid discharge part and a target skin and to place the plural needles therein. Front end portions of the plural needles uniformly protrude from the front end portion of the outer case.

The outer case includes a combining groove formed on the inner circumferential surface of a rear end portion thereof and the inner case includes a combining protrusion formed on the outer circumferential surface thereof, so that the outer case and the inner case are combined together by combination of the combining protrusion and the combining groove.

The outer case and the inner case are combined together by an adhesive.

The combining groove includes: a vertical combining groove formed logitudinally from the rear end portion of the outer case; and a horizontal combining groove which extends from the vertical combining groove and is formed in a rotational direction of the inner circumferential surface of the outer case.

The medicinal fluid discharge part further includes: through holes formed at the front end portion of the outer case so that the plural needles penetrate the through holes; a connection part 522 for connecting rear end portions of the plural needles located inside the outer case with one another; and elastic members which are respectively fit to the plural needles located inside the outer case, each of the elastic members having one end which is supported on one side of the connection part and the other end which is supported on the inner surface of the outer case so as to provide the plural needles with an elastic force.

The medicinal fluid placing part includes: a motor which pressurizes the storage container so as to supply the medicinal fluid from the storage container to the medicinal fluid supply part at a predetermined ratio per hour, wherein the medicinal fluid is repeatedly and consecutively supplied within a range of 50 µℓ to 250 µℓ at a time; and a control part for controlling operation of the motor.

The plural needles are arranged in a row.

The medicinal fluid injection device further includes: an air pressure supplying part for supplying air pressure; a control part for guiding the air pressure supplied from the air pressure supplying part to the medicinal fluid pressurization part; and a switch part which is connected to the control part to control supply of the air pressure.

The diameter of the through hole is within a range of 50 µm to 125 µm, and the medicinal fluid pressurization part is operated by an air compressor or compressed gas to supply pressure of 1 bar to 10 bar.

### Advantageous Effects

As described above, the medicinal fluid injection device according to the present invention can induce generation of collagen to obtain skin reproduction effect because the medicinal fluid passes through the epidermis and reaches the dermal layer in the form of a microjet by pressure.

Moreover, the medicinal fluid injection device according to the present invention can minimize an injection loss rate within the skin when the microjet permeates through the skin and remove a patient's resistance due to pain and bleeding.

Furthermore, the medicinal fluid injection device according to the present invention can transfer the medicinal fluid of a uniform amount to a uniform depth in response to the skin characteristics of an object when the medicinal fluid having active constituents with various material properties.

Additionally, the medicinal fluid injection device according to the present invention has the improved structure to endure high pressure applied to the medicinal fluid, thereby solving the problem of the fluid loss.

### Description of Drawings

FIGS. 1 and 2 are schematic views showing a medicinal fluid injection device according to a first preferred embodiment of the present invention.
FIGS. 3 and 4 are exploded perspective views of a medicinal fluid discharge part according to the first preferred embodiment of the present invention.
FIG. 5 is a sectional view of a medicinal fluid discharge part according to a second preferred embodiment of the present invention.
FIG. 6 is a schematic view showing a medicinal fluid supply part according to the preferred embodiment of the present invention.

### Mode for Invention

The following descriptions are provided to assist in a comprehensive understanding of an embodiment of the present invention. Well known techniques, elements, structures, and processes will be omitted to avoid obscuring the subject matter of the present invention.

The terms or words used in the following description and the claims are not limited to conventional or dictionary meanings and should be interpreted as having meanings and definitions suitable for the technical sprit of the invention based on a principle that the inventor(s) may suitably define terms to describe the invention in a best mode.

Therefore, the description of elements stated in the specification and shown in the drawings has been made only for a better understanding of the present invention, and those skilled in the art will appreciate that various modifications, additions, and substitutions to the specific elements are possible, without departing from the scope and spirit of the invention as disclosed in the accompanying claims.

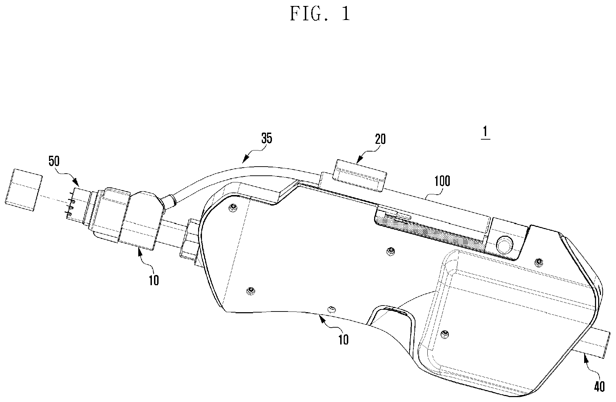

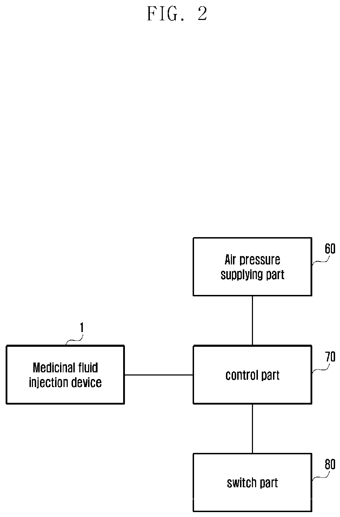

FIGS. 1 and 2 are schematic views showing a medicinal fluid injection device according to a first preferred embodiment of the present invention.

Referring to FIG. 1, the medicinal fluid injection device 1 according to a first preferred embodiment of the present invention includes a case part 10, a medicinal fluid placing part 20, a medicinal fluid supply part 30, a medicinal fluid pressurization part 40 and a medicinal fluid discharge part 50.

The case part 10 is to form the outward appearance of a product and is formed in such a way that a user can grasp it with the hand. For instance, the case part 10 is made from plastic resin and a plurality of case parts 10 are assembled together.

The medicinal fluid placing part 20 is mounted on the case part 10, and a storage container 100 in which medicinal fluid is stored is mounted.

The medicinal fluid supply part 30 is connected with the storage container 100 to supply the medicinal fluid from the storage container 100 to the medicinal fluid discharge part, so that the medicinal fluid stored in the storage container 100 flows in.

The medicinal fluid pressurization part 40 is mounted on the case part 10, and is connected to the medicinal fluid supply part 30.

The medicinal fluid pressurization part 40 supplies pressure to the medicinal fluid supply part 30 to pressurize the medicinal fluid supplied to the medicinal fluid supply part 30. The medicinal fluid pressurization part 40 may be operated by an air compressor or compressed gas so as to provide pressure of 1 bar to 10 bar.

The medicinal fluid discharge part 50 is mounted on the medicinal fluid supply part 30 to spout the medicinal fluid.

The medicinal fluid discharge part 50 is arranged near to the skin, and the medicinal fluid spouting from the medicinal fluid discharge part 50 reaches the dermal layer after passing through the epidermis.

The medicinal fluid injection device 1 according to the preferred embodiment of the present invention further includes an air pressure supplying part 60, a control part 70 and a switch part 80.

The air pressure supplying part 60 is connected to the control part 70 to supply air pressure, and the control part 70 is connected with the medicinal fluid pressurization part 40 to guide air pressure.

The switch part 80 is connected to the control part 70, and transfers an on-off signal to the control part 70 according to the user's pressurization.

Therefore, the user grasps the case part 10 to locate near to the skin, and then, manipulates the switch part 80. When the user operates the switch part 80, the control part 70 transfers the air pressure of the air pressure supplying part 60 to the medicinal fluid pressurization part 40.

When the air pressure is supplied, the medicinal fluid pressurization part 40 pressurizes the medicinal fluid stored in the medicinal fluid supply part 30, and the medicinal fluid pressurized in the medicinal fluid supply part 30 passes through the medicinal fluid discharge part 50 at high velocity and at high pressure and infiltrates into the epidermis, and then, is deteriorated in velocity and pressure to spread in the dermal layer in a radial form.

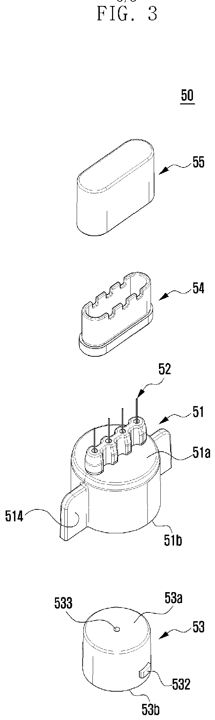

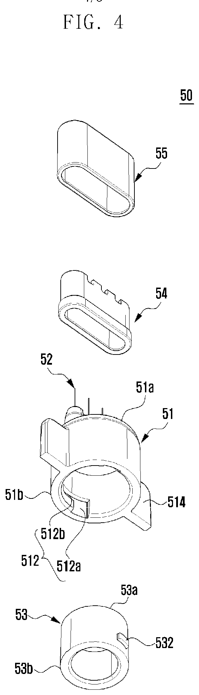

FIGS. 3 and 4 are exploded perspective views of a medicinal fluid discharge part according to the first preferred embodiment of the present invention.

The medicinal fluid discharge part 50 includes an outer case 51 and an inner case 53.

A plurality of needles 52 are mounted at a front end portion 51a of the outer case. 53 is mounted at a rear end portion 51b of the outer case 51.

The inner case 53 is mounted when the front end portion 53a is inserted and joined into the outer case 51 and the rear end portion 53b is joined to the medicinal fluid supply part 30.

A through hole 533 which penetrates through the inner case 53 is formed at the front end portion 53a of the inner case 53.

The medicinal fluid is formed into a microjet by the through hole 533. When the medicinal fluid passes the through hole 533 by high pressure, the medicinal fluid is discharged in the form of the microjet.

The microjet formed in the through hole is supplied to the inside of the skin by the needle 52.

The through hole 533 may be formed to have the desired diameter in order to form the microjet.

According to a preferred embodiment of the present invention, when the diameter of the through hole 533 is regulated, a jet force of the microjet can be controlled. Because the diameter of the through hole 533 is in inverse proportion to the jet force of the microjet, the diameter of the through hole 533 is regulated according to the skin of an object or areas and properties of tissues so as to control the jet force of the microjet.

According to a preferred embodiment of the present invention, preferably, the diameter of the through hole 533 is within a range of 50 µm to 125 µm. If the diameter of the through hole 533 is shorter than 50 µm, it is difficult to form the microjet, but if the diameter of the through hole 533 exceeds 125 µm, the microjet may be converted into fluid and discharged in the form of fluid.

According to a preferred embodiment of the present invention, an injected area can be controlled. In more detail, in order to control the injected area of the microjet, a plurality of the needles 52 are arranged within a predetermined area in order to control the injected area. The conventional injection needle can inject medicinal fluid only at one point when it is inserted into the skin once. However, the medicinal fluid discharge part 50 according to the preferred embodiment of the present invention can inject medicinal fluid at a number of points over the desired area of the target skin because a plurality of the needles 52 are arranged over a predetermined area. Therefore, the medicinal fluid injection device according to the present invention can inject the medicinal fluid over the desired area at a time.

The plural needles 53 are formed to inject the medicinal fluid into the target skin tissues. The microjet discharged through the through hole 533 may be guided to the previously set depth of the skin by the plural needles 52. According to the preferred embodiment of the present invention, for the plural needles 53, needles of a micro-size may be used.

According to the preferred embodiment of the present invention, the needles 52 come into contact with the skin before injection in such a way as to penetrate through the dead skin cells or the epidermis and to be located on the upper part of the dermal layer. Moreover, the microjet of high-velocity and high-pressure is discharged under the dermal layer at high velocity and infiltrates to a target area so as to spread and be absorbed to surrounding tissues.

The conventional needle-free device is difficult to infiltrate the microjet under the dermal layer because directly injecting the microjet to the dead skin cells or the epidermis. Moreover, the conventional needle-free device reduces efficiency of the minimum invasion injection device because the microjet cannot permeate the dead skin cells or the epidermis and is reflected in the reverse direction. However, according to the preferred embodiment of the present invention, because a plurality of the needles 52 guide the microjet to the dead skin cells or the epidermis, the microjet can permeate the dead skin cells or the epidermis in safety and can be injected to the desired depth. Especially, even though the dead skin cells and the epidermis are different in thickness and intensity by objects and by parts of objects, the fluid injection device can easily inject the microjet under the dermal layer because a plurality of the needles 52 guide the microjet to the dead skin cells and the epidermis without adjusting velocity and pressure of the microjet.

Furthermore, the conventional syringe is difficult to adjust the insertion depth of the needle, causes a patient's pain and bleeding due to a hole formed in the skin by the needle, and also causes a loss of the medicinal fluid because the medicinal fluid is discharged out through the needle hole formed in the skin. However, the medicinal fluid injection device according to the present invention almost never cause the patient's pain and bleeding and solves the problem that causes a loss of the medicinal fluid due to the discharge of the medicinal fluid because a plurality of the needles 52 penetrate into the skin to the minimum.

The insertion depth of the needles 52 according to the preferred embodiment of the present invention is within a range of 0.1mm to 20mm. If the insertion depth of the needles 52 is less than 0.1mm, it is difficult that the needles 52 pass through the dead skin cells or epidermis to guide the medicinal fluid, but if the insertion depth of the needles 52 is more than 20mm, the patient may feel pain.

Preferably, the insertion depth of the needles 52 may be within a range of 0.5mm to 10mm and within a range of 0.2mm to 5mm according to the position of the target area and the thickness of the skin.

The inner diameter of each of the needles 52 according to the present invention may be less than 0.5mm according to the properties, such as viscosity, of the medicinal fluid or the target area. Especially, the minimum diameter of the needle is less than 0.3mm, preferably, less than 0.2mm, so that the medicinal fluid receives pressure inside the medicinal fluid discharge part 50 so as to quickly permeate into the desired skin tissues.

A plurality of the needles may be arranged in a row. When the plural needles are arranged in a row, the medicinal fluid can extensively permeate into the target wrinkled skin along the length direction.

The medicinal fluid discharge part 50 can be disassembled from the case part 10. Therefore, the medicinal fluid discharge part 50 can be replaced with a new one according to patients. That is, the needles can be used more hygienically because the medicinal fluid discharge part 50 is replaced with a new one.

In order to form the microjet, high pressure must be applied to the through hole 533.

However, when high pressure is applied to the medicinal fluid, it may cause a leakage of the medicinal fluid at a connection part between the components according to a flowing path of the medicinal fluid. In order to solve the above problem, the medicinal fluid discharge part 50 includes the outer case 51 and the inner case 53 and has the following features.

The outer case 51 has a combining groove 512 formed on the inner circumferential surface of the rear end portion 51b of the outer case 51.

The inner case 53 has a combining protrusion 532 formed on the outer circumferential surface thereof.

When the combining protrusion 532 and the combining groove 512 are combined together, the outer case 51 and the inner case 53 are combined with each other.

Moreover, the outer case 51 and the inner case 53 are completely combined with each other by an adhesive. Of course, the outer case 51 and the inner case 53 may be also combined by screw-coupling, and in this instance, it is preferable that they be completely sealed through the adhesive in order to prevent the leakage of the medicinal fluid.

While the microjet formed from the medicinal fluid of high pressure is guided into the target skin through the through hole 533 by the needles 52, pressure of the microjet in a space between the outer case 51 and the inner case 53 is kept to be less than pressure of the medicinal fluid inside the inner case 53. Therefore, the high pressure applied to the medicinal fluid is not applied to the needles 52.

Due to the dual case of the outer case 51 and the inner case 53, even though the high pressure is applied to the medicinal fluid, pressure of the medicinal fluid is not transferred to the outer case 51 and a leakage of the medicinal fluid and a damage of the needles by pressure of the medicinal fluid can be prevented.

The combining groove 512 includes a vertical combining groove 512a formed in the length direction from the rear end portion 51b of the outer case and a horizontal combining groove 512b formed in a rotational direction of the inner circumferential surface of the outer case 51.

In order to insert the inner case 53 into the rear end portion 51b of the outer case 51, the combining protrusion 532 must be located at the vertical combining groove 512a. That is, in a state where the combining protrusion 532 is combined to the combining groove 512a, when the inner case 53 is pushed into the outer case 51, the combining protrusion 532 moves along the vertical combining groove 512a. After that, when the inner case 53 is rotated relative to the outer case 51, the combining protrusion 532 moves along the horizontal combining groove 512b. In the above state, the inner case 53 and the outer case 51 are not separated from each other in the length direction of a rotary shaft.

The inner case 53 is screw-coupled to the medicinal fluid supply part 30.

A hand-grip plate 514 may be formed on the outer circumferential surface of the outer case 51. The hand-grip plate 514 protrudes out of the outer case 51 so that the user can grasp the medicinal fluid discharge part 50 with the hand. The user can smoothly rotate the medicinal fluid discharge part 50 through the hand-grip plate 514.

The medicinal fluid discharge part 50 includes a spacing part 54. The spacing part 54 is to uniformly maintain a distance between the medicinal fluid discharge part 50 and the target skin. A plurality of the needles 52 are located inside the spacing part 54. The spacing part 54 is detachably mounted on the front end portion 51a of the outer case 51. The length of the spacing part 54 is adjustable so that the needles 52 project out from the front end portion 51a of the outer case 51. So, spacing parts 54 with different lengths are prepared previously. A front end portion of the spacing part 54 gets in contact with the target skin, and a rear end portion of the spacing part 54 is combined with the outer case 51. The spacing part 54 uniformly maintains the distance between the outer case 51 and the target skin. Additionally, the spacing part 54 uniformly maintains the depth that the needles are inserted into the target skin.

It is preferable that the depth that the needles 52 are inserted into the skin be adjustable within a range of 0.1mm to 20mm. If the insertion depth of the needles 52 is 0.1mm, it is difficult that the needles 52 pass through the dead skin cells or the epidermis to guide the microjet. If the insertion depth of the needles 52 exceeds 20mm, the patient may feel pain. Preferably, the depth of the needles 52 is adjustable within the range of 0.1mm to 20mm. For this, the needles 52 may be manufactured in various lengths, but it is very complicated and it is difficult to replaceably mount on the outer case 51. In order to solve the above problem, the spacing part 54 which is simply replaceable is mounted. The spacing part 54 is detachably combined to the front end portion 51a of the outer case 51. The spacing parts 54 which have pieces of different heights are prepared previously to be replaced with another one, so that the depth of the needle 52 which is inserted into the skin can be adjusted.

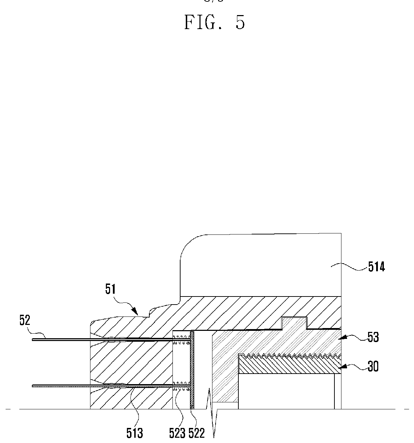

FIG. 5 is a sectional view of a medicinal fluid discharge part 50 according to a second preferred embodiment of the present invention.

The medicinal fluid discharge part 50 includes: through holes 513 formed at the front end portion 51a of the outer case 51 so that the plural needles 52 penetrate the through holes; a connection part 522 for connecting rear end portions of the plural needles 52 located inside the outer case 51 with one another; and elastic members 523 which are respectively fit to the needles 52 located inside the outer case 51 and of which one end is supported on one side of the connection part 522 and the other end is supported on the inner surface of the outer case 51 so as to provide the plural needles with an elastic force.

In more detail, the medicinal fluid discharge part has a plurality of through holes 513 formed at the front end portion 51a of the outer case 51.

The plural needles 52 respectively penetrate the plural through holes 513. The rear end portions of the needles 52 located inside the outer case 51 are connected with one another by the connection part 522.

The needles 52 get the elasticity of the elastic member 523 in the direction that they are inserted into the outer case 51 when pressure of the medicinal fluid is not applied to the inside of the outer case 51, but the front end portion of the needles 52 protrudes outwardly from the spacing part 54 by pressure of the medicinal fluid when pressure of the medicinal fluid is applied to the inside of the outer case 51. The reason is to make the needles 52 reciprocate to a predetermined length in the state where the needles 52 protrude out from the outer case 51. When pressure is not applied to the medicinal fluid, the plural needles 52 are located inside the spacing part 54, but, when pressure is applied to the medicinal fluid, the needles advance toward the skin by pressure of the microjet located between the outer case 51 and the inner case 53. Moreover, the tip portion of each needle 52 protrudes outwardly from the spacing part 54. The length that the needle 52 protrudes from the spacing part 54 may be set previously.

The medicinal fluid placing part 20 includes: a motor (not shown) which pressurizes the storage container 100, in which the medicinal fluid is stored, so as to supply the medicinal fluid from the storage container 100 to the medicinal fluid supply part 30 at a predetermined ratio per hour, wherein the medicinal fluid is repeatedly and consecutively supplied within a range of 50 µℓ to 250 µℓ at a time; and a control part (not shown) for controlling operation of the motor. The storage container 100 in which the medicinal fluid is stored is mounted on the medicinal fluid placing part 20. The medicinal fluid placing part 20 pressurizes the storage container 100 to supply the medicinal fluid from the storage container into the medicinal fluid supply part 30. The storage container 100 and the medicinal fluid supply part 30 are connected with each other by a connection hose 35.

Because the needles 52 are inserted into the skin and reach the dermal layer when the needles 52 protrude above the spacing part 54, the needles 52 must uniformly keep the distance between the needles 52 and the target skin by the spacing part 54.

A needle cover 55 is combined to the outer case 51 to cover the front end portions of the needles 52, thereby keeping the needles 52 clean and enhancing safe-keeping of the needles 52.

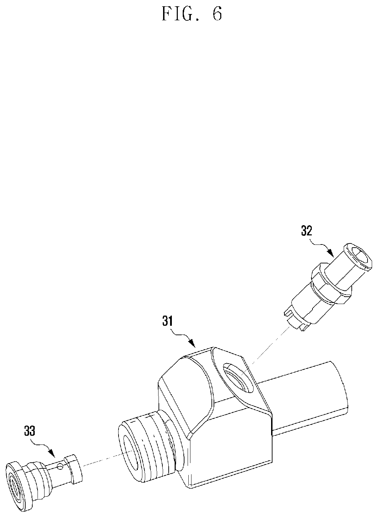

FIG. 6 is a schematic view showing the medicinal fluid supply part 30 according to the preferred embodiment of the present invention.

The medicinal fluid supply part 30 includes a medicinal fluid storing part 31, a check valve part 32 and a pressure valve part 33.

The medicinal fluid storing part 31 is mounted on the case part 10 and forms a space (not shown) in which the medicinal fluid is stored. The pressure valve part 33 is inserted into the medicinal fluid storing part 31.

The check valve part 32 is mounted on the medicinal fluid storing part 31 and connected to the storage container 100 to guide the medicinal fluid. The check valve part 32 passes the medicinal fluid in one direction by a general check valve. That is, the medicinal fluid stored in the storage container 100 is moved to the medicinal fluid storing part 31 by the check valve part 32, but the medicinal fluid stored in the medicinal fluid storing part 31 does not move to the storage container 100.

The pressure valve part 33 is built in the medicinal fluid storing part 31, and passes the medicinal fluid in one direction by a pressure difference.

The inner case 53 is screw-coupled with the medicinal fluid supply part 30. The rear end portion 53b of the inner case 53 is screw-coupled with the outer surface of the front end portion of the medicinal fluid storing part 31.

As described above, while the present invention has been particularly shown and described with reference to the specific embodiments thereof, it will be understood by those of ordinary skill in the art that the above embodiments of the present invention are all exemplified and the present invention is not limited to the above embodiment. It also will be understood by those of ordinary skill in the art that various changes in form and details may be made therein without departing from the spirit and scope of the present invention as defined by the following claims.

## Claims

1. A medicinal fluid injection device, which injects medicinal fluid into the skin in such a way as to radially spread the medicinal fluid so as to promote collagen generation of the skin, comprising:
a case part which forms an outward appearance;
a medicinal fluid placing part, which is mounted on the case part, and, to which a storage container storing medicinal fluid therein is mounted;
a medicinal fluid supplying which is connected with the storage container to supply the medicinal fluid;
a medicinal fluid pressurization part which is mounted on the case part and is connected to the medicinal fluid supply part to pressurize the supplied medicinal fluid; and
a medicinal fluid discharge part which is mounted on the medicinal fluid supply part to inject the medicinal fluid supplied from the medicinal fluid supply part into the skin,
wherein the medicinal fluid discharge part includes:
an outer case on which a plurality of needles are mounted; and
an inner case of which a front end portion is inserted and combined into the outer case and a rear end portion is combined and mounted to the medicinal fluid supply part; and
a through hole is formed at the front end portion of the inner case in such a way as to penetrate through the inner case in order to form a microjet.

2. The medicinal fluid injection device according to claim 1, wherein the medicinal fluid discharge part further includes a spacing part which is detachably mounted at a front end portion of the outer case in order to uniformly maintain a distance between the medicinal fluid discharge part and a target skin and to place the plural needles therein, and
wherein front end portions of the plural needles uniformly protrude from the front end portion of the outer case.

3. The medicinal fluid injection device according to claim 2, wherein the outer case includes a combining groove formed on the inner circumferential surface of a rear end portion thereof and the inner case includes a combining protrusion formed on the outer circumferential surface thereof, so that the outer case and the inner case are combined together by combination of the combining protrusion and the combining groove.

4. The medicinal fluid injection device according to claim 3, wherein the outer case and the inner case are combined together by an adhesive.

5. The medicinal fluid injection device according to claim 4, wherein the combining groove includes: a vertical combining groove formed longitudinally from the rear end portion of the outer case; and
a horizontal combining groove which extends from the vertical combining groove and is formed in a rotational direction of the inner circumferential surface of the outer case.

6. The medicinal fluid injection device according to claim 5, wherein the medicinal fluid discharge part further includes:
through holes formed at the front end portion of the outer case so that the plural needles penetrate the through holes;
a connection part for connecting rear end portions of the plural needles located inside the outer case with one another; and
elastic members which are respectively fit to the plural needles located inside the outer case, each of the elastic members having one end which is supported on one side of the connection part and the other end which is supported on the inner surface of the outer case so as to provide the plural needles with an elastic force.

7. The medicinal fluid injection device according to claim 6, wherein the medicinal fluid placing part includes: a motor which pressurizes the storage container so as to supply the medicinal fluid from the storage container to the medicinal fluid supply part at a predetermined ratio per hour, wherein the medicinal fluid is repeatedly and consecutively supplied within a range of 50 µℓ to 250µℓ at a time; and a control part for controlling operation of the motor.

8. The medicinal fluid injection device according to claim 7, wherein the plural needles are arranged in a row.

9. The medicinal fluid injection device according to claim 8, further comprising:
an air pressure supplying part for supplying air pressure;
a control part for guiding the air pressure supplied from the air pressure supplying part to the medicinal fluid pressurization part; and
a switch part which is connected to the control part to control supply of the air pressure.

10. The medicinal fluid injection device according to claim 9, wherein the diameter of the through hole is within a range of 50µm to 125µm, and
wherein the medicinal fluid pressurization part is operated by an air compressor or compressed gas to supply pressure of 1 bar to 10 bar.
